(19) Europäisches Patentamt
European Patent Office
Office européen des brevets

(11) **EP 2 513 324 B1**

(12) **EUROPÄISCHE PATENTSCHRIFT**

(45) Veröffentlichungstag und Bekanntmachung des Hinweises auf die Patenterteilung:
**13.07.2016 Patentblatt 2016/28**

(51) Int Cl.:
*C12P 19/04* (2006.01)     *C08B 37/00* (2006.01)
*B01D 61/14* (2006.01)     *B01D 63/06* (2006.01)
*B01D 65/02* (2006.01)

(21) Anmeldenummer: **10790953.3**

(22) Anmeldetag: **13.12.2010**

(86) Internationale Anmeldenummer:
**PCT/EP2010/069518**

(87) Internationale Veröffentlichungsnummer:
**WO 2011/082973 (14.07.2011 Gazette 2011/28)**

(54) **VERFAHREN ZUR HERSTELLUNG VON HOMOPOLYSACCHARIDEN**

METHOD FOR PRODUCING HOMOPOLYSACCHARIDES

PROCÉDÉ DE PRÉPARATION D'HOMOPOLYSACCHARIDES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **17.12.2009 EP 09179716**
**17.12.2009 US 287224 P**

(43) Veröffentlichungstag der Anmeldung:
**24.10.2012 Patentblatt 2012/43**

(73) Patentinhaber: **Wintershall Holding GmbH**
**34119 Kassel (DE)**

(72) Erfinder:
• **THERRE, Jörg**
**67551 Worms (DE)**
• **VOSS, Hartwig**
**67227 Frankenthal (DE)**
• **SCHMIDT, Julia Kristiane**
**69115 Heidelberg (DE)**
• **FAUST, Tillmann**
**67256 Weisenheim am Sand (DE)**
• **HOLLMANN, Rajan**
**49152 Bad Essen (DE)**

(74) Vertreter: **BASF IP Association**
**BASF SE**
**ZRX-C6**
**67056 Ludwigshafen (DE)**

(56) Entgegenhaltungen:
**WO-A2-03/016545     DE-A1- 4 012 238**

• **Rau U: "Schizophyllan", Biopolymers online, 15. Januar 2005 (2005-01-15), Seiten 1-20, XP002648709, Gefunden im Internet: URL:http://onlinelibrary.wiley.com/doi/10. 1002/3527600035.bpol6003/full [gefunden am 2011-07-06]**
• **DATABASE BIOSIS [Online] BIOSCIENCES INFORMATION SERVICE, PHILADELPHIA, PA, US; 2000, RAU U ET AL: "Production, downstream processing and characteristics of fungal beta-glucans (Schizophyllan, Scleroglucan)", XP002227192, gefunden im BIOSIS Database accession no. PREV200100093721**
• **SONDHI R ET AL: "Applications and benefits of ceramic membranes", MEMBRANE TECHNOLOGY, Bd. 2003, Nr. 11, November 2003 (2003-11), Seiten 5-8, XP002648710, DOI: 10.1016/S0958-2118(03)11016-6**
• **Luque S et al.: "Industrial Applications of Porous Ceramic Membranes (Pressure Driven Processes)" In: MALLADA R & MENÉNDEZ M (EDS): "Inorganic membranes: Synthesis, characterization and applications", 1. Januar 2008 (2008-01-01), BOOK SERIES: MEMBRANE SCIENCE AND TECHNOLOGY, ELSEVIER B V, XP008139269, ISBN: 978-0-444-53070-7 Seiten 177-216, [gefunden am 2008-04-04] Seiten 201-203, Absatz 3.4.**

• Anonymous: "Pall® Membralox® ceramic membranes and modules", Pall Corporation, Datasheet, Juli 2007 (2007-07), Seiten 1-2, XP002648711, Gefunden im Internet: URL:http://web.archive.org/web/20100102085 033/http://pall.com/pdf/PIMEMBRAEN.pdf [gefunden am 2011-07-06]

• Anonymous: "Pall® Membralox® GP ceramic membranes with longitudinal permeability gradient", Pall Corporation, Datasheet, Juli 2007 (2007-07), Seiten 1-2, XP002648712, Gefunden im Internet: URL:http://www.pall.com/pdf/PIMEMBRAGPEN. p df [gefunden am 2011-07-06]

**Beschreibung**

Die vorliegende Erfindung betrifft ein Verfahren zur Herstellung von wässrigen Lösungen von Glucanen mit einer $\beta$-1,3-glykosidisch verknüpften Hauptkette sowie $\beta$-1,6-glykosidisch daran gebundenen Seitengruppen durch Fermentation von Pilzstämmen, welche die genannten Glucane in die Fermentationsbrühe sekretieren, in einem wässrigen Nährmedium wobei die Abtrennung der Glucane aus der Fermentationsbrühe unter Verwendung asymmetrischer Filtermembranen erfolgt.

In natürlichen Erdölvorkommen liegt Erdöl in den Hohlräumen poröser Speichergesteine vor, welche zur Erdoberfläche hin von undurchlässigen Deckschichten abgeschlossen sind. Bei den Hohlräumen kann es sich um sehr feine Hohlräume, Kapillaren, Poren oder dergleichen handeln. Feine Porenhälse können beispielsweise einen Durchmesser von nur ca. 1 $\mu$m aufweisen. Neben Erdöl, inklusive Anteilen von Erdgas enthält eine Lagerstätte mehr oder weniger stark salzhaltiges Wasser.

Bei der Erdölförderung unterscheidet man zwischen der primären, sekundären und tertiären Förderung.

Bei der primären Förderung strömt das Erdöl nach dem Anbohren der Lagerstätte aufgrund des Eigendrucks der Lagerstätte von selbst durch das Bohrloch an die Oberfläche. Mittels der primären Förderung lassen sich je nach Lagerstättentyp aber meist nur ca. 5 bis 10% der in der Lagerstätte vorhandenen Erdölmenge fördern, danach reicht der Eigendruck nicht mehr zur Förderung.

Nach der primären Förderung kommt daher die sekundäre Förderung zum Einsatz. Bei der sekundären Förderung werden zusätzlich zu den Bohrlöchern, welche der Förderung des Erdöls dienen, den sogenannten Produktionsbohrungen, weitere Bohrlöcher in die erdölführende Formation gebohrt. Durch diese sogenannten Injektionsbohrungen wird Wasser und/oder Dampf in die Lagerstätte eingepresst, um den Druck aufrechtzuerhalten oder wieder zu erhöhen. Durch das Einpressen des Wassers wird das Erdöl durch die Hohlräume in der Formation langsam von der Injektionsbohrung ausgehend in Richtung der Produktionsbohrung gedrückt. Dies funktioniert aber nur so lange, wie die Hohlräume vollständig mit Öl gefüllt sind und das viskosere Öl durch das Wasser vor sich her geschoben wird. Sobald das dünnflüssige Wasser durch Hohlräume durchbricht, strömt es ab diesem Zeitpunkt auf dem Weg des geringsten Widerstandes, also durch den gebildeten Kanal zwischen den Injektions- und den Produktionsbohrungen und schiebt nicht mehr das Öl vor sich her. Mittels primärer und sekundärer Förderung sind im Regelfalle nur ca. 30 bis 35 % der in der Lagerstätte vorhandenen Erdölmenge zu fördern.

Es ist bekannt, die Erdölausbeute durch Maßnahmen der tertiären Ölförderung weiter zu steigern. Zur tertiären Erdölförderung gehören Verfahren, bei denen man geeignete Chemikalien als Hilfsmittel zur Ölförderung einsetzt. Hierzu gehört das so genannte "Polymerfluten". Beim Polymerfluten presst man durch die Injektionsbohrungen anstelle von Wasser eine wässrige Lösung eines verdickend wirkenden Polymers in die Erdöllagerstätte ein. Durch das Einpressen der Polymerlösung wird das Erdöl durch die besagten Hohlräume in der Formation von der Injektionsbohrung ausgehend in Richtung der Produktionsbohrung gedrückt, und das Erdöl wird schließlich über die Produktionsbohrung gefördert. Aufgrund der hohen Viskosität der Polymerlösung, welche an die Viskosität des Erdöls angepasst wird, kann die Polymerlösung nicht mehr oder zumindest nicht so leicht durch Hohlräume durchbrechen wie dies bei reinem Wasser der Fall ist.

Zum Polymerfluten sind eine Vielzahl verschiedener wasserlöslicher Polymere vorgeschlagen worden, und zwar sowohl synthetische Polymere, wie beispielsweise Polyacrylamide oder Acrylamid und andere Monomere enthaltende Copolymere sowie auch wasserlösliche Polymere natürlichen Ursprungs.

Geeignete verdickend wirkende Polymere für die tertiäre Erdölförderung müssen eine Reihe von speziellen Anforderungen erfüllen. Neben einer ausreichenden Viskosität müssen die Polymere auch sehr temperaturstabil sein und ihre verdickende Wirkung auch bei hohen Salzkonzentrationen behalten.

Eine wichtige Klasse von Polymeren natürlichen Ursprungs für das Polymerfluten bilden verzweigte Homopolysaccharide aus Glucose. Polysaccharide aus Glucoseeinheiten werden auch Glucane genannt. Die genannten verzweigten Homopolysaccharide weisen eine Hauptkette aus $\beta$-1,3-verknüpften Glucoseeinheiten auf, von denen -statistisch gesehen etwa jede dritte Einheit mit einer weiteren Glucoseeinheit $\beta$-1,6-glykosidisch verknüpft ist. Wässrige Lösungen derartiger verzweigter Homopolysaccharide besitzen vorteilhafte physikalisch-chemische Eigenschaften, so dass sie für das Polymerfluten besonders gut geeignet sind.

Homopolysaccharide der genannten Struktur werden von verschiedenen Pilzstämmen sekretiert, beispielsweise von dem filamantös wachsenden Basidiomycet Schizophyllum commune, der während des Wachstums ein Homopolysaccharid der genannten Struktur mit einem typischen Molekulargewicht $M_w$ von ca. 5 bis ca. $25*10^6$ g/mol ausscheidet (Trivialname Schizophyllan). Weiterhin zu nennen sind von Sclerotium rolfsii sekretierte Homopolysaccharide der genannten Struktur (Trivialname: Scleroglucane).

Wichtig für das Polymerfluten ist es, dass die hierzu verwendete wässrige Polymerlösung keinerlei Gelpartikel oder sonstige kleine Partikel enthält. Bereits eine geringe Anzahl von Partikeln mit Dimensionen im Mikrometerbereich können die feinen Poren in der Erdölformation verstopfen und somit die Erdölförderung zumindest erschweren oder sogar zum Erliegen bringen. Polymere zur tertiären Erdölförderung sollten daher einen möglichst geringen Anteil an Gelpartikeln oder sonstigen kleinen Partikeln aufweisen.

Für die Verwendung zum Polymerfluten ist es daher wichtig, dass Lösungen der genannten Homopolysaccharide weitgehend frei von Zellen und Zellbruchstücken sind, da diese sonst die Erdölformation verstopfen können, was die Gewinnung des Erdöls erschwert oder sogar unmöglich macht. Als Kenngröße für eine gute Qualität einer Polymerlösung kann das sogenannte Millipore-Filtrations-Verhältnis (Millipore filtration ratio; MPFR-Wert) verwendet werden. Hierbei wird bestimmt, wie sich der Filterwiderstand beim Filtrieren einer Lösung im Laufe der Zeit ändert.

Verfahren zur Herstellung von verzweigten Homopolysacchariden aus β-1,3-verknüpften Glucoseeinheiten sind bekannt.

EP 271 907 A2, EP 504 673 A1 und DE 40 12 238 A1 offenbaren Verfahren zur Herstellung, und zwar erfolgt die Herstellung durch diskontinuierliche Fermentation des Pilzes Schizophyllum commune unter Rühren und Belüftung. Das Nährmedium besteht im Wesentlichen aus Glucose, Hefe-Extrakt, Kaliumdihydrogenphoshat, Magnesiumsulfat und Wasser. EP 271 907 A2 beschreibt eine Methode zur Isolierung der Polysaccharids, bei der man die Kultursuspension zunächst zentrifugiert und das Polysaccharid aus dem Überstand mit Isopropanol ausfällt. Eine zweite Methode umfasst eine Druckfiltration gefolgt von einer Ultrafiltration der erhaltenen Lösung, ohne dass Details zur Methode offenbart sind.

"Udo Rau, "Biosynthese, Produktion und Eigenschaften von extrazellulären Pilz-Glucanen", Habilitationsschrift, Technische Universität Braunschweig, 1997, Seiten 70 bis 95" beschreibt die Herstellung von Schizophyllan durch kontinuierliche oder diskontinuierliche Fermentation. Die Abtrennung des Schizophyllans kann mittels Querstromfiltration erfolgen (a.a.O, S. 75). Zur Abtrennung der Zellmasse wurden verschiedene Edelstahlmembranen mit Porenweiten von 0,5 μm, 2 μm, 10 μm und 20 μm getestet. Mit 2 μm-Membranen wurden jedoch mit einer Lösung, welche 0,5 g/l Glucan und 0,5 g/l Biotrockenmasse enthielt, nur geringe Permeationsraten erhalten. Außerdem verblieben Hyphenfragmente in einer Konzentration von ca. 0,1 g/ml. Es wird daher ein zweiter Feinstklärungsschritt vorgeschlagen (a.a.O. S. 94). Ein derartiges Verfahren ist sehr aufwändig, und außerdem sind Edelstahl-Membranen sehr teuer.

"Udo Rau, Biopolymers, Hrsg A. Steinbüchel, Volume 6, Seiten 63 bis 79, Verlag WILEY-VCH, New York, 2002 " beschreibt die Herstellung von Schizophyllan durch kontinuierliche oder diskontinuierliche Fermentation. Zur Gewinnung des zell- und zellbruchstückfreien Schizophyllans wird in die Zentrifugation und die Querstrom-Mikrofiltration empfohlen (a.a.O., S. 78, Abs. 10.1). Für die Querstrom-Mikrofiltration wird dort der Einsatz von gesinterten Edelstahl-Membranen mit einer Porengröße von 10 μm vorgeschlagen. Das so erhaltene Permeat muss aber nochmals mittels Diafiltration gereinigt werden sowie ggf. mittels Querstrom-Mikrofiltration aufgereinigt werden (a.a.O., S. 78, Abs. 10.2). Ein derartiges Verfahren ist sehr aufwändig, und außerdem sind Edelstahl-Membranen sehr teuer.

In "GIT Fachzeitung Labor 12/92 Seite 1233 - 1238" wird eine kontinuierliche Herstellung verzweigter β-1,3-Glucane mit Zellrückführung beschrieben. Zur Abtrennung der verzweigten β-1,3-Glucane aus dem Fermentationskreislauf wird zunächst eine Querstromfiltration mittels Edelstahlmembranen vorgeschlagen, die eine Porengröße von 200 μm besitzen. Das erhaltene polymerhaltige Permeat ist aber noch mit großen Mengen an Zellbruchstücken verunreinigt, und muss in einem zweiten Schritt nachgereinigt werden. Hierzu werden eine Tiefenfiltration mit einem Glasfasertiefenfilter, eine Dreistufendruckfiltration sowie die Zentrifugation vorgeschlagen. Als weitere Methode für die zweite Reinigungsstufe haben die Autoren ohne Erfolg die Querstromfiltration an keramischen Membranen untersucht. Als Ergebnis aus ihren Experimenten ziehen sie den Schluss, dass die Querstrom-Mikrofiltration nicht für die Zellseparation von mycelhaltigen, hochviskosen Kultursuspensionen geeignet ist. Das erhaltene Permeat wird schließlich in einer dritten Reinigungsstufe mittels Diafiltration nachgereinigt. Ein derartiger dreistufiger Prozess ist aber sehr aufwändig, und dementsprechend für einen industriellen Herstellprozess ungeeignet.

WO 03/016545 A2 offenbart ein kontinuierliches Verfahren zur Herstellung von Scleroglucanen unter Verwendung von Sclerotium rolfsii. Zur Reinigung wird eine Querstromfiltration unter Verwendung von Edelstahlfiltern einer Porengröße von 20 μm bei einer Überströmgeschwindigkeit von mindestens 7 m/s beschrieben. Ein 20 μm-Filter ist jedoch zu Abtrennung sehr kleiner Partikel nicht ausreichend.

Die Abtrennung feiner Partikel könnte zwar im Prinzip durch die Verwendung feinerer Filtermembranen verbessert werden. Mit abnehmender Porengröße halten die Filtermembranen aber in unerwünschter Art und Weise zunehmend auch die Glucane zurück, insbesondere die Fraktionen mit sehr hohen Molekulargewichten. Weiterhin erfordern feinere Membranen höhere Filterdrücke und damit nimmt die Gefahr zu, dass der Pilz mechanisch zu stark belastet werden könnte. Es sollte vermieden werden, dass Zellen zerstört werden und lysieren, denn dadurch wird das herzustellende Polymer verunreinigt.

Weiterhin sollte aus wirtschaftlichen Gründen die Konzentration erhaltener wässriger Glucan-Lösungen möglichst hoch sein, und zwar einerseits um möglichst kleine Fermentationsanlagen verwenden zu können, und andererseits um einen möglichst geringen Transportaufwand zu haben, um die wässrigen Glucan-Lösungen von der Produktionsstätte zum Einsatzort zu transportieren. Es sollte aus wirtschaftlichen Gründen eine Konzentration von mindestens 3 g/l Glucan angestrebt werden. Glucan-Lösungen mit einer derart hohen Konzentration weisen eine sehr hohe Viskosität auf und sind darüber hinaus stark strukturviskos. Derartige Lösungen sind schwierig zu filtrieren. Je höher die Konzentration, desto schwieriger ist der Filtrationsschritt.

Aufgabe der vorliegenden Erfindung war es, ein wirtschaftliches Verfahren zur Herstellung von Lösungen

# EP 2 513 324 B1

verzweigter β-1,3-Glucane bereitzustellen, wobei die Lösungen eine zur Verwendung in der tertiären Erdölförderung ausreichende Qualität haben sollten. Die Lösungen sollten neben einer hohen spezifischen Viskosität insbesondere einen möglichst niedrigen Gehalt an Zellen und Zellbruchstücken aufweisen. Mit den Filtraten sollten Spezifikationswerte der Filtrierbarkeit MPFR < 2,5 mit 1,2 μm Isopore-Filtern erreicht werden. Dem entsprechend wurde ein Verfahren zur Herstellung von wässrigen Lösungen von Glucanen mit einer β-1,3-glykosidisch verknüpften Hauptkette sowie β-1,6-glykosidisch daran gebundenen Seitengruppen gefunden, wobei das Verfahren die Fermentation von Pilzstämmen, welche Glucane der genannten Struktur sekretieren, in einem wässrigen Nährmedium und anschließender Abtrennung einer wässrigen Lösung des gebildeten Glucans aus der wässrigen, Glucane und Biomasse enthaltenden Fementationsbrühe durch Querstrom-Mikrofiltration umfasst, wobei die Konzentration der Glucane in der zu filtrierenden Fermentationsbrühe mindestens 3 g/l beträgt, und wobei für die Querstrom-Mikrofiltration asymmetrische Filtermembranen umfassend mindestens eine Schicht aus einem Trägermaterial und mindestens einer Trennschicht eingesetzt werden, wobei die Porengröße der Trennschicht 1 μm bis 10 μm und die Porengröße des Trägermaterials 5 μm bis 100 μm beträgt, mit der Maßgabe, dass die Porengröße des Trägermaterials mindestens 1 μm größer ist als die Porengröße der Trennschicht, und wobei die Strömungsgeschwindigkeit der Querströmung 0,2 m/s bis 20 m/s und der Transmembrandruck 0,1 bis 10 bar beträgt. Verzeichnis der Abbildungen

Abbildung 1: Schematische Darstellung einer bevorzugten Filtrationsapparatur.

Abbildung 2: Schematische Darstellung der für die Versuche und Vergleichsversuche verwendeten Apparatur.

Zu der Erfindung ist im Einzelnen das Folgende auszuführen:

Unter "Glucanen" versteht der Fachmann Homopolysaccharide, welche ausschließlich aus Glucoseeinheiten aufgebaut sind. Mittels des erfindungsgemäßen Verfahrens wird eine spezielle Klasse von Glucanen hergestellt, und zwar solche, die eine Hauptkette aus β-1,3-glykosidisch verknüpften Glucoseeinheiten sowie β-1,6-glykosisch daran gebundenen Seitengruppen aus Glucoseeinheiten umfassen. Bevorzugt bestehen die Seitengruppen aus einer einzigen β-1,6-glykosidisch angebundenen Glucoseeinheit, wobei -statistisch gesehenjede dritte Einheit der Hauptkette mit einer weiteren Glucoseeinheit β-1,6-glykosisch verknüpft ist.

Derartige Glucane sekretierende Pilzstämme sind dem Fachmann bekannt. Beispiele umfassen Schizophyllum commune, Sclerotium rolfsii, Sclerotium glucanicum, Monilinia fructigena, Lentinula edodes oder Botrytis cinera. Geeignete Pilzstämme sind weiterhin beispielsweise in EP 271 907 A2 sowie EP 504 673 A1, jeweils Anspruch 1, genannt. Bevorzugt handelt es sich bei den eingesetzten Pilzstämmen um Schizophyllum commune oder Sclerotium rolfsii und besonders bevorzugt um Schizophyllum commune, welcher ein Glucan sekretiert, bei dem an eine Hauptkette aus β-1,3-glykosidisch verknüpften Glucoseeinheiten -statistisch gesehen- jede dritte Einheit der Hauptkette mit einer weiteren Glucoseeinheit β-1,6-glykosisch verknüpft ist; d.h. bevorzugt handelt es sich bei dem Glucan um das so genannte Schizophyllan. Typische Schizophyllane weisen ein gewichtsmittleres Molekulargewicht $M_w$ von ca. 5 bis ca. 25*10^6 g/mol auf.

In einem ersten Verfahrensschritt werden die Pilze in einem geeigneten wässrigen Nährmedium fermentiert. Die Pilze sekretieren im Zuge der Fermentation die oben genannte Klasse von Glucanen in die wässrige Fermentationsbrühe.

Verfahren zur Fermentation derartiger Pilzstämme sind dem Fachmann prinzipiell bekannt, beispielsweise aus EP 271 907 A2, EP 504 673 A1, DE 40 12 238 A1, WO 03/016545 A2 sowie "Udo Rau, "Biosynthese, Produktion und Eigenschaften von extrazellulären Pilz-Glucanen", Habilitationsschrift, Technische Universität Braunschweig, 1997", welche jeweils auch geeignete Nährmedien nennen.

Erfindungsgemäß können die Pilze beispielsweise in einem wässrigen Nährmedium bei einer Temperatur von 15°C bis 40°C, bevorzugt 25 bis 30°C und beispielsweise ca. 27°C, bevorzugt unter Belüftung und Bewegung, beispielsweise mit einem Rührer, kultiviert werden.

Beim erfindungsgemäßen Verfahren wird die Fermentationen so gefahren, dass die Konzentration der herzustellenden Glucane in der zu filtrierenden Fermentationsbrühe mindestens 3 g/l beträgt. Die Obergrenze ist nicht prinzipiell beschränkt. Sie ergibt sich daraus, welche Viskosität mit der jeweils verwendeten Fermentationsapparatur noch handhabbar ist.

Aus der Fermentationsbrühe, welche gelöste Glucane sowie Biomasse (pilzliche Zellen sowie ggf. Zellbestandteile) enthält, wird schließlich eine wässrige, Glucane enthaltende Lösung durch Querstrom-Mikrofiltration abgetrennt, wobei eine wässrige Fermentationsbrühe verbleibt, in der die Biomasse eine höhere Konzentration aufweist als vorher.

In einer Ausführungsform des Verfahrens wird die Fermentation in einem Fermentationsbehälter durchgeführt und der Inhalt des Fermentationskessels nach der Fermentation erfindungsgemäß unter Verwendung asymmetrischer Filtermembranen filtriert. In einer weiteren Ausführungsform der Erfindung wird die Fermentation in einer geeigneten Anlage, welche mindestens einen Fermentationsbehälter umfasst, durchgeführt. Der Anlage wird über einen Seitenstrom ständig oder zeitweise Fermentationsbrühe entnommen und daraus eine wässrige, Glucane enthaltende Lösung durch Querstrom-Mikrofiltration abgetrennt. Die verbliebene wässrige Fermentationsbrühe, in der die Biomasse eine höhere Konzentration aufweist als vorher, kann zumindest zu einem Teil wieder in den Fermentationsbehälter zurückgeführt.

Das Verfahren der Querstrom-Mikrofiltration ist dem Fachmann prinzipiell bekannt und wird zum Beispiel in "Melin, Rautenbach, Membranverfahren, Springer-Verlag, 3. Auflage, 2007, Seite 309 bis Seite 366" beschrieben. Unter "Mikrofiltration" versteht der Fachmann hierbei, die Abtrennung von Partikeln einer Größe zwischen ca. 0,1 $\mu$m bis ca.10 $\mu$m.

Bei der Querstrom-Filtration, auch Cross-Flow-Filtration genannt, wird -beispielsweise durch eine geeignete Umwälzpumpe- eine Strömung der zu filtrierenden Flüssigkeit parallel zur Oberfläche der als Filtrationsmaterial eingesetzten Membran aufgebracht. Die Filtermembran wird also ständig von einem Flüssigkeitsstrom überströmt, und hierdurch wird die Bildung von Ablagerungen auf der Membranoberfläche verhindert oder zumindest vermindert. Als Pumpe sind prinzipiell alle Pumpentypen geeignet. Wegen der hohen Viskosität des zu fördernden Mediums haben sich aber insbesondere Verdrängerpumpen und ganz besonders Exzenterschneckenpumpen und Kreiskolbenpumpen bewährt.

Erfindungsgemäß werden zur Querstrom-Mikrofiltration asymmetrische Filtermembranen eingesetzt. Asymmetrische Filtermembranen bestehen aus mindestens zwei verschiedenen Schichten mit unterschiedlicher Porengröße, und zwar aus mindestens einer Trägerschicht und einer Trennschicht. Die Trägerschicht ist vergleichsweise dick und hat vergleichsweise große Poren. Sie verleiht der Filtermembran die mechanische Festigkeit. Auf der Trägerschicht ist mindestens eine Trennschicht mit feineren Poren als die Poren der Trägerschicht aufgebracht. Zur Messung der Porengrößen kann in prinzipiell bekannter Art und Weise beispielsweise die Quecksilber-Porosimetrie verwendet werden. Optional können zwischen der Trennschicht und der Trägerschicht noch eine oder mehrere Zwischenschichten angeordnet werden.

Bei den asymmetrischen Membranen kann es sich beispielsweise um metallische Membranen oder um keramische Membranen handeln. Bevorzugt handelt es sich bei den eingesetzten asymmetrischen Membranen um asymmetrische keramische Membranen. Einzelheiten zu asymmetrischen keramischen Membranen sind beispielsweise in "Melin, Rautenbach, Membranverfahren, Springer-Verlag, 3. Auflage, 2007, Seite 51 bis Seite 52" beschrieben.

Aus dem Trägermaterial ist der Körper der keramischen oder metallischen Membran hergestellt. Geeignete Formen dieser Membrankörper sind dem Fachmann bekannt und werden vom Fachmann je nach der Konstruktion der Filterapparatur gewählt. Sie können beispielsweise als Flachmembran oder Rohrmembran ausgebildet sein. Flachmembranen stellen dabei scheibenförmige Gebilde dar. Rohrmembranen sind röhrenförmige Gebilde, die einen Kanal (Einkanalmembran) oder mehrere Kanäle (Mehrkanalmembran) besitzen. Der Innendurchmesser der Kanäle von Rohrmembranen beträgt in der Regel 1 mm bis 25 mm, insbesondere 2 mm bis 12,5 mm. Die Kanäle müssen nicht rund sein, sondern es sind auch unregelmäßige Formen, wie beispielsweise Vielecke mit abgerundeten Ecken möglich. Die Rohrmembranen sind in der Regel zwischen 0,1 m und 5 m lang, bevorzugt 0,5 bis 2 m. Handelsüblich sind Rohrmembranen von 1 m bis 1,2 m Länge. Es können auch mehrere Rohrmembranen ggf. auch in unterschiedlichen Gehäusen, sogenannten Membranmodulen hintereinander oder parallel zueinander angeordnet werden.

Das Trägermaterial besteht bei keramischen Filtermembranen aus einem porösen anorganischen Material wie beispielsweise Aluminiumoxid, Siliziumoxid, Siliziumkarbid, Zirkonoxid, Titanoxid oder Mischungen dieser Stoffe. Bei metallischen Membranen wird Sintermetall, wie beispielsweise Edelstahl, Hastelloy, Inconell oder Titan als Trägermaterial verwendet. Es sind auch Materialkombinationen möglich, beispielsweise von Sintermetallträgern und keramischen Trennschichten. Bei Einkanalmembranen oder Flachmembranen ist das Trägermaterial zwischen in der Regel 0,05 bis 10 mm dick, bevorzugt zwischen 1 mm bis 5 mm.

Besonders bevorzugt ist die Verwendung von Mehrkanalmembranen. Bei Mehrkanalmembranen bildet das Trägermaterial einen Formkörper, beispielsweise einen runden oder sechseckigen Formkörper, in den die oben genannten Kanäle eingelassen sind. Der Außendurchmesser eines derartigen Formkörpers für Mehrkanalmembranen beträgt in der Regel 5 mm bis 100 mm, bevorzugt 10 mm bis 50 mm.

Beim erfindungsgemäßen Verfahren zur Herstellung von Glucanen mit einer β-1,3-glykosidisch verknüpften Hauptkette sowie β-1,6-glykosidisch daran gebundenen Seitengruppen Lösung beträgt die Porengröße des Trägermaterials 5 $\mu$m bis 100 $\mu$m, bevorzugt 7 $\mu$m bis 100 $\mu$m und besonders bevorzugt 10 $\mu$m bis 60 $\mu$m.

Bei den genanten Werten handelt es sich jeweils um die Porengröße D90. Der Begriff "Porengröße D90" ist dem Fachmann bekannt. Er wird aus einer Porengrößenverteilungskurve des Trägermaterials ermittelt, wobei die "Porengröße D90" diejenige Porengröße bezeichnet, bei der 90 % des Porenvolumens des Materials eine Porengröße $\leq$ Porengröße D90 aufweisen. Die Porengrößenverteilung eines Materials kann beispielsweise mittels Quecksilberporosimetrie und/oder Gasadsoptionsmethoden bestimmt werden. Diese Methoden sind dem Fachmann prinzipiell bekannt und beispielsweise in den einschlägigen Normen ISO 15901-1 EN, ISO 15901-2 EN sowie ISO 15901-3 EN beschrieben.

Auf das Trägermaterial können optional eine oder mehrere Zwischenschichten aufgebracht sein. Auf der Trägerschicht oder auf optional vorhandenen Zwischenschichten folgt eine Trennschicht. Die mittlere Porengröße der Trennschicht beträgt 1 bis 10 $\mu$m, bevorzugt 1 $\mu$m bis 6 $\mu$m und besonders bevorzugt 2 $\mu$m bis 5 $\mu$m. Bei den Werten handelt es sich wie oben beschrieben um D90 Porengrößen.

Die Porengrößen der Trägerschicht und der Trennschicht werden vom Fachmann jeweils so gewählt, dass die Porengröße der Trägerschicht mindestens 1 μm größer ist als die der Trennschicht. Bevorzugt ist die die Porengröße der Trägerschicht mindestens 5 μm größer als die der Trennschicht, besonders bevorzugt mindestens 10 μm und beispielsweise mindestens 20 μm.

Die Trennschicht und die Zwischenschichten können beispielsweise aus Aluminiumoxid, Siliziumoxid, Siliziumkarbid, Zirkonoxid, Titanoxid, Mischungen dieser Stoffe oder aus Metalllegierungen bestehen. Es ist nicht erforderlich, dass die Trennschicht, die Zwischenschichten und das Trägermaterial aus den gleichen Stoffen hergestellt werden, oft ist gerade die Kombination von verschiedenen Stoffen vorteilhaft.

Die Dicke der optional vorhandenen Zwischenschichten beträgt zwischen 1 μm und 500 μm. Die durchschnittliche Dicke der Trennschicht beträgt in der Regel 1 μm bis 50 μm bevorzugt 5 μm bis 200 μm. Die Zwischenschichten besitzen Porengrößen, die zwischen der jeweils gewählten Porengröße des Trägermaterials und der Porengröße der Trennschicht liegen.

Zur Durchführung des erfindungsgemäßen Verfahrens werden die asymmetrischen Filtermembranen in geeignete Filterapparaturen eingebaut. Konstruktionen geeigneter Filterapparaturen sind dem Fachmann prinzipiell bekannt. Es ist vorteilhaft, wenn sich die Trennschicht zwischen Trägermaterial und Retentatraum befinden, ohne dass die Erfindung darauf beschränkt ist.

Für die Durchführung des erfindungsgemäßen Verfahrens können bevorzugt Rohrmembranen verwendet werden. Bei Rohrmembranen wird das Retentat bevorzugt durch das Innere des Kanals oder der Kanäle geleitet, und das Permeat tritt dem entsprechend nach außen durch die Wände des Trägermaterials in den Permeatraum aus. Weniger bevorzugt ist es, wenn sich Retentat außerhalb des Kanals oder der Kanäle befindet, und sich das Permeat im Innern des Kanals oder der Kanäle sammelt.

Die Rohrmembranen können als sogenannte Monokanalelemente eingesetzt werden. Bevorzugt ist aber die Verwendung von Mehrkanalelementen. Diese Elemente bieten den Vorteil der größeren Membranfläche bei gleichem Platzbedarf, der einfacheren Montage und damit der deutlich niedrigeren Investitionskosten. Bei diesen Membranelementen muss aber das Permeat den gesamten Stützkörper durchdringen, um aus dem Membranelement auszutreten. Bei strukturviskosen Stoffen ist die Viskosität bei niedrigen Strömungsgeschwindigkeiten besonders hoch, was den Durchtritt einer Glucanlösung durch den Stützkörper erschwert. Daher war zur vermuten, dass wegen des langen Weges und des erschwerten Abflusses des Permeates durch den Stützkörper, Mehrkanalelemente nicht für die Filtration von Schizophyllan-Lösungen geeignet sein könnten.

Es wurde aber gefunden, dass trotz der hochviskosen und strukturviskosen Eigenschaft des Permeates der Einsatz von Mehrkanalelementen möglich ist und selbst bei niedrigen Transmembrandrücken hohe Permeatflüsse erreicht werden können.

Erfindungsgemäß sollte die Strömungsgeschwindigkeit der Querströmung 0,2 m/s bis 20 m/s betragen, bevorzugt 0,5 m/s bis 7 m/s und besonders bevorzugt 1 m/s bis 6 m/s. Eine zu niedrige Strömungsgeschwindigkeit ist ungünstig, da die Membran dann schnell verstopft, eine zu hohe Strömungsgeschwindigkeit verursacht wegen der großen umzuwälzenden Menge an Retentat unnötig hohe Kosten.

Der Transmembrandruck beträgt in der Regel 0,1 bar bis 10 bar, bevorzugt 0,5 bar bis 6 bar, und ganz bevorzugt 1 bar bis 4 bar.

Die Temperatur, bei der die Querstrom-Mikrofiltration durchgeführt wird, ist nicht kritisch und liegt in der Regel zwischen 5°C und 150°C, bevorzugt 10 bis 80°C und besonders bevorzugt 15 bis 40°C. Wenn die abgetrennten Zellen nicht abgetötet werden sollen, also beispielsweise bei Verfahren mit Rückführung der Biomasse, sollte die Temperatur 15°C bis 40°C betragen.

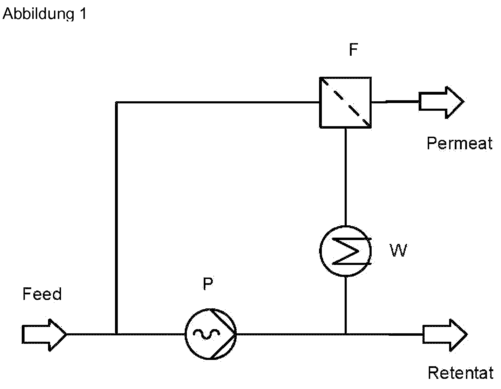

Eine bevorzugte Ausführungsform einer erfindungsgemäß zu verwendenden Filtereinheit ist in Abbildung 1 dargestellt. Die bevorzugte Apparatur umfasst eine Umwälzpumpe P, ein Filtermodul F und einen Wärmetauscher W. Mittels der Pumpe P wird die oben erwähnte Querströmung der Flüssigkeit über die Oberfläche der im Filterapparat F angeordneten Membran erzeugt. Mit einem Wärmetauscher W kann der Anlageninhalt temperiert werden.

Der Filterapparat F besteht aus einem Gehäuse, in dem eine Membran als Trennwand eingebracht ist. Durch die Membran wird das Gehäuse in einen sogenannten Retentatraum und einen Permeatraum getrennt. Die von der Pumpe P kommende Flüssigkeit, Feed genannt, ist die Glucan-Lösung, die mit Biomasse verunreinigt ist. Der Feed tritt über mindestens einen Zulauf in den Retentatraum ein. Durch mindestens einen Ablauf tritt ein Flüssigkeitsstrom, Konzentrat genannt, wieder aus dem Retentatraum aus. Der Druck im Retentatraum ist höher als der Druck im Permeatraum. Die Druckdifferenz wird Transmembrandruck genannt. Ein Teil des Feedstromes tritt durch die Membran hindurch und sammelt sich im Permeatraum. Dieser hindurch tretende Teil der Flüssigkeit, Permeat genannt, stellt die von Biomasse getrennte Glucan-Lösung dar.

In einer weiteren Ausführungsform der Erfindung können hohe Scherkräfte über der Membranoberfläche erhalten werden, indem man rotierende Einbauten verwendet oder die Membran an sich rotiert. In diesem Fall spricht man auch von dynamischer Cross-Flow-Filtration. Apparaturen zur Durchführung einer dynamischen Cross-Flow-Mikrofiltration sind dem Fachmann bekannt, und können beispielsweise unter dem Namen DynaMem-Modul der Fa. Buss-SMS-Cancler GmbH, Düren, erworben werden. Bei der Verwendung einer solchen dynamischen Cross-Flow-Mikrofiltrationsapparatur werden die beschriebenen asymmetrischen Keramikmembranen in Scheibenform eingesetzt.

Die Betriebszeit der Membranfiltrationsanlage kann optional durch eine regelmäßige Rückspülung mit Permeat verlängert werden. Dazu wird in regelmäßigen Abständen im Permeatraum ein Druck angelegt, der höher ist als der Druck im Retentatraum und für eine definierte Zeit eine bestimmte Menge an Permeat rückwärts durch die Membran in den Retentatraum zurückgedrückt. Dieses Rückspülen kann beispielsweise durch das Aufpressen des Permeatraumes mit Stickstoff, durch eine Rückspülpumpe oder durch das Verwenden eines Kolbensystems erfolgen, wie es z.B. unter der Bezeichnung "BACKPULSE DECOLMATEUR BF 100" von der Firma Pall, Bad Kreuznach, vertrieben wird. Die Rückspülung sollte im Abstand von 1 Minute bis zu 5 Stunden erfolgen, bevorzugt im Abstand von 2 Minuten bis 60 Minuten, ohne dass die Erfindung auf diesen Zeittakt beschränkt sein soll. Die Menge an zurückspülten Permeat liegt bevorzugt im Bereich von 0,05 bis 5 Liter pro $m^2$ Membranfläche, bevorzugt aber im Bereich von 0,1 bis 2 Liter pro $m^2$ Membranfläche.

Je nach der Qualität des eingesetzten Fermentationsaustrages kann es erforderlich sein, die verwendeten Filtermembranen zu gegebener Zeit zu reinigen. Die Reinigung der Filtermembranen kann erfolgen, indem man die Membranen mit einer geeigneten Reinigungslösung bei einer Temperatur von 20°C bis 100°C, insbesondere von 40°C bis 80°C behandelt. Als Reinigungslösung können Säuren (Mineralsäuren wie beispielsweise Phosphorsäure, Salpetersäure oder organische Säuren wie beispielsweise Ameisensäure) verwendet werden. Die Säurekonzentration liegt in der Regel bei einer Konzentration von 1 Gew. % bis 10 Gew. %. Bessere Reinigungswirkungen werden in der Regel durch den Einsatz von Laugen (z.B. Natronlauge, Kalilauge) erzielt. Die Konzentration verwendeter Laugen liegt zwischen 0,1 Gew. % und 20 Gew. %. Durch die Zugabe von oxidierend wirkenden Stoffen, wie beispielsweise Wasserstoffperoxid, Hypochlorit, insbesondere Natriumhypochlorit oder Peressigsäure kann die Reinigungswirkung deutlich verbessert werden. Die Konzentration der oxidierend wirkenden Stoffe sollte 0,5 Gew. % bis 10 Gew. % betragen, insbesondere 1 Gew. % bis 5 Gew. % . Besonders bevorzugt kann die Reinigung mit einer Mischung aus Wasserstoffperoxid und Lauge oder Wasserstoffperoxid und Hypochlorit vorgenommen werden. Die Reinigung der Membranen erfolgt -bei abgestellter Anlage- bevorzugt im in der Membranfiltrationsanlage eingebauten Zustand mit Hilfe eines Cleaning-in-Place-Systems (CIP-System). Es hat sich bewährt, die Reinigung der Filtermembranen vorzunehmen, sobald eine Permeatmenge von 50 kg pro $m^2$ Membranfläche bis zu 5000 kg pro $m^2$ Membranfläche erhalten worden ist, bevorzugt 50 kg pro $m^2$ Membranfläche bis zu 1000 kg pro $m^2$.

Mittels des erfindungsgemäßen Verfahrens kann eine für die tertiäre Erdölförderung geeignete Lösung von Glucanen mit einer $\beta$-1,3-glykosidisch verknüpften Hauptkette sowie $\beta$-1,6-glykosidisch daran gebundenen Seitengruppen auf einfache Art hergestellt werden.

Die erfindungsgemäß verwendeten asymmetrischen Membranen sind preiswert. Die Membrananlage erfordert wegen der hohen Permeatflüsse niedrige Investitionskosten und hat einen niedrigen Energieverbrauch. Die asymmetrischen Membranen weisen hohe Standzeiten auf.

Die gute Qualität des Produktes zeigt sich in den guten Filtrationseigenschaften, die durch das niedrige Filtrations-Verhältnis (MPFR-Wert) ausgedrückt werden. Der MPFR-Wert des Produktes liegt zwischen 1,001 und 2,5, insbesondere aber zwischen 1,01 und 2,0.

Die Ausbeute an Schizophyllan, also die Menge an Schiziphyllan, die aus dem Fermenationsaustrag gewonnen werden kann bezogen auf die Menge des im Fermenteraustrages vorliegenden Menge an Schizophylan, liegt zwischen 25% und 97%, insbesondere zwischen 30% und 95% und ganz besonders zwischen 50% und 93%.

Durch das dem Fachmann bekannte Verfahren einer Diafiltration mit Wasser kann die Ausbeute an Glucan gegebenenfalls gesteigert werden.

Die folgenden Beispiele sollen die Erfindung näher illustrieren:

Bestimmung des Filtrations-Verhältnis (MPFR-Wert)

Messprinzip:

Bei der Bestimmung des Millipore-Filtrations-Verhältnis (Millipore filtration ratio; MPFR-Wert) wird die Menge des Filtrats, welche durch einen definierten Filter läuft, in Abhängigkeit der Zeit bestimmt. Der MPFR-Wert wird gemäß folgender Formel (I) bestimmt

$$\text{MPFR} = (t_{190g} - t_{170g}) \, / \, (t_{70g} - t_{50g}) \quad \text{(I),}$$

wobei die Variablen in der Gleichung die folgende Bedeutung haben:

$t_{190g}$ = Zeit, in der 190 g Filtrat erhalten werden,

$t_{170g}$ = Zeit, in der 170 g Filtrat erhalten werden,

$t_{70g}$ = Zeit, in der 70 g Filtrat erhalten werden,

$t_{50g}$ = Zeit, in der 50 g Filtrat erhalten werden.

Es wird also jeweils die Zeitspanne bestimmt, welche für den Durchfluss von jeweils 20 g Filtrat benötigt werden, und zwar zu einem frühen Zeitpunkt und zu einem späten Zeitpunkt des Filtrationsvorganges, und aus den beiden Zeitspannen wird der Quotient gebildet. Je größer der MPFR-Wert, umso stärker verlangsamt sich die Filtrationsgeschwindigkeit mit zunehmender Dauer des Filtrationsvorganges. Dies deutet auf eine zunehmende Verstopfung des Filters, beispielsweise durch Gele oder Partikel hin.

Der MPFR-Wert wird nach folgender Vorschrift bestimmt:

1. Ausrüstung

a) Satorius-Druckfiltrationsgerät 16249; Filterdurchmesser 47 mm; mit Aufgusszylinder 220 mL ($\varnothing$i = 41 mm)

b) Isopore Membran 1,2 $\mu$m; 0 47 mm; Nr. RTTP04700

c) Waage

2. Ansetzen der Glucanlösung

Es wird zunächst 50 g einer Mischung der aus den Versuchen erhaltenen Glucanlösung sowie Reinstwasser angesetzt, und zwar in einem solchen Verhältnis, dass die Konzentration des Glucans 1,75 g/l beträgt. Die Mischung wird 10 min gerührt und visuell auf Homogenität geprüft. Sofern die Mischung noch nicht homogen ist, wird weitergerührt bis die Mischung homogen ist. Anschließend wird die Mischung mit 200 g Reinstwasser auf eine Gesamtmenge von 250 g aufgefüllt. Anschließend wird mindestens 1 h zur Homogenisierung gerührt, danach der pH-Wert mit 0,1 m NaOH auf 6,0 eingestellt und anschließend nochmals 15 min gerührt. Der pH-Wert von 6,0 wird nochmals geprüft. Die Endkonzentration des Glucans in der Mischung beträgt 0,35 g/l.

3. Versuchsdurchführung Filtration

Der Filtrationstest erfolgt bei Raumtemperatur (T = 25°C) bei einem Druck von 1,0 bar (Druckluft oder $N_2$).

- Grobes Stützgitter auf den Siebboden legen
- Feines Stützgitter auf den Siebboden legen
- Membranfilter auflegen
- Dichtung (O-Ring) einlegen
- Siebboden und Auslasshahn am Zylinder verschrauben
- Auslasshahn verschließen
- 220 g (ca. 220ml) Lösung einfüllen
- Oberen Deckel mit Zylinder verschrauben
- Zuluftschlauch anklemmen
- Druck überprüfen und auf 1,0 bar einstellen
- Becherglas auf die Waage unter der Filtrationsapparatur stellen. Tara drücken.
- Auslasshahn öffnen
- Der Versuch wird abgebrochen, wenn kein Filtrat mehr austritt.

Mittels der Waage wird die Menge des Filtrats in Abhängigkeit der Zeit bestimmt. Die jeweils angezeigte Masse kann visuell aber selbstverständlich auch automatisch abgelesen und ausgewertet werden.

Rückhaltung:

Zur Charakterisierung des Trennverhaltens der Membran wird die Rückhaltung R verwendet (siehe Melin, Rautenbach, a.a.O., Seite 6).

R = 1 - (Konzentration an Glucan im Permeat) zu einem Zeitpunkt dividiert durch die Konzentration Glucan im Retentat zu diesem Zeitpunkt.

Da das Glucan als Permeat gewonnen wird, sollte die Rückhaltung so gering wie möglich sein. Bei einer Mikrofiltration die Rückhaltung ist in der Regel größer als 0 %. Da sich die Rückhaltung während der Zeit ändern kann, wird als Kenngröße eine mittlere Rückhaltung über die Zeit angegeben.

Mit den erfindungsgemäß verwendeten Filtermembranen werden mittlere Rückhaltungen von unter 60%, in günstigen Fällen sogar unter 30% erhalten. Dies bedeutet, dass das Glucan weitgehend aus der Fermentationsbrühe gewonnen werden kann.

Aufkonzentrierfaktor:

Bei der Aufkonzentrierung der Fermentationsbrühe ist der Aufkonzentrierungsfaktor MK eine wichtige Größe. Er ist definiert als das Verhältnis der Masse der eingesetzten Fermentationsbrühe zum Zeitpunkt Null dividiert durch die Masse der Fermentationsbrühe am Ende der Glucanabtrennung. Der Aufkonzentrierungsfaktor soll möglichst groß sein.

Mit dem erfindungsgemäßen Verfahren können Aufkonzentrierungsfaktoren bis zu 15, in günstigen Fällen sogar bis 30 erreicht werden.

Vergleichsbeispiel

Filtration mit einer symmetrischen Filtermembran

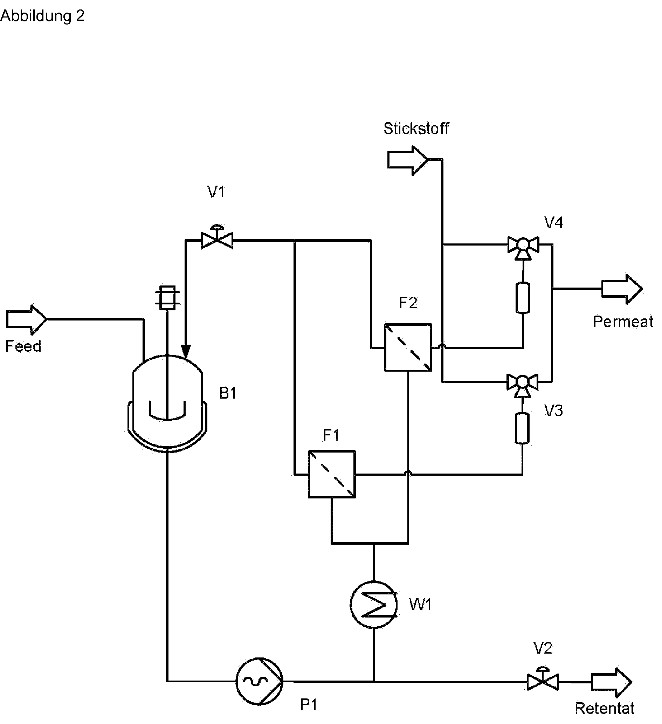

Die verwendete Querstrom-Filtrationsapparatur ist in der Abbildung 2 dargestellt. Sie bestand aus einem gerührten Doppelmantel-Vorlagebehälter B1 mit einem Volumen von 120 Liter, der Exzenterschneckenpumpe P1, dem Rohrbündel-Wärmetauscher W1, dem Druckhalteventil V1 und den beiden Filtermodulen F1 und F2. Die Filtermodule F1 und F2 wurden mittels der Dreiwegehähne V3 und V4 mit Permeat im Abstand von jeweils 300 s mit jeweils 200 ml Permeat zurückgespült, der Druck des Stickstoffes betrug 7 bar. Über den Doppelmantel des Behälters B1 und den Wärmetauscher W1 wurde der Inhalt der Querstromfiltrationsanlage auf 24°C gekühlt.

In den Filtermodulen F1 und F2 wurde eine symmetrische Rohrmembran eingesetzt, und zwar ein 5-Kanal-Element der Firma TAMI aus der Keramik ATZ (Alumina/Titania/Zirconia).

Die Porengröße D90 der Membran betrug 3,5 $\mu$m. Die Membran war symmetrisch aufgebaut und besaß keine Trennschicht oder Zwischenschichten. Die Länge des Membranrohres war 1 m, der Außendurchmesser war 20 mm. Die Membranfläche eines Modulelementes betrug 0,11 m$^2$. Der hydraulische Durchmesser eines Kanals betrug 6 mm.

Für die Versuche wurde Schizophyllum commune verwendet, und zwar wurde das Schizophyllan wie in "Udo Rau, Biopolymers, Hrsg A. Steinbüchel, Verlag WILEY-VCH, Volume 6, Seiten 63 bis 79" beschrieben in einer Batch-Fermentation hergestellt. Die Fermentationszeit betrug 96 Stunden. 99,6 kg dieser Fermentationsbrühe (=Feed) wurde in den Behälter B1 (Abb. 2) eingefüllt und 45 Minuten lang bei 4 bar Druck mit einer Umwälzmenge von 7 m$^3$/h mittels der Pumpe P1 umgewälzt. Der Inhalt des Behälters wurde analysiert und ein Gehalt von 9,8 Gramm Schizophyllan pro Liter ermittelt.

Dann wurde die Umwälzmenge auf 5,1 m$^3$/h eingestellt und ein Transmembrandruck von 1,1 bar angelegt. Die Überströmgeschwindigkeit war 5 m/s. Das aus den Filtermodulen austretende Permeat wurde gesammelt und gewogen. Während der ersten 10 Minuten des Experimentes wurden 0,75 kg Permeat erhalten. Dies entspricht einem Permeatfluss von 20,4 kg/h/m2. Der Transmembrandruck war 2,9 bar. Die Filtration wurde 16 Stunden lang betrieben und in dieser Zeit 6,18 kg Permeat erhalten. Innerhalb der letzten Stunde konnten nur noch 5,4 g Permeat gewonnen werden, da die Membranen nahezu vollständig verstopft waren.

Das gesammelte Permeat wurde analysiert und ein Glucangehalt von 6,7 Gramm pro Liter gefunden. Die Ausbeute betrug also nur 4 %. Der MPFR-Wert des Permeates betrug 2,8 und die mittlere Rückhaltung an Glucan während des Experimentes war 32 %. Der Aufkonzentrierungsfaktor war nur 1,07.

Erfindungsgemäßes Beispiel 1

Filtration mit einer asymmetrischen Filtermembran

Es wurde wiederum die im Beispiel 1 beschriebene Querstrom-Filtrationsapparatur verwendet. Die Filtermodule F1 und F2 wurden mittels der Dreiwegehähne V3 und V4 mit Permeat im Abstand von jeweils 120 s mit jeweils 200 ml Permeat zurückgespült, der Druck des Stickstoffes betrug 4 bar. Über den Doppelmantel des Behälters B1 und den Wärmetauscher W1 wurde der Inhalt der Querstromfiltrationsanlage auf 22°C gekühlt.

In den Filtermodulen F1 und F2 wurde eine asymmetrische Rohrmembran aus SIC eingesetzt, und zwar ein 37-Kanal-Element (Modell "CRYSTAR , Typ FT 3000" der Fa. St. Gobain). Die Porengröße D90 der Membranen betrug 3,0 $\mu$m. Die Porengröße D90 des Trägermaterials betrug 30 $\mu$m. Die Länge des Membranrrohres war 1 m, der Außendurchmesser war 32 mm. Die Membranfläche eines Modulelementes betrug 0,42 m$^2$. Der hydraulische Durchmesser

eines Kanals war 3,4 mm.

Für den Versuche wurde der im Bespiel 1 beschriebene Fermentationsaustrag verwendet. 115 kg dieser Fermentationsbrühe (=Feed) wurde in den Behälter B1 eingefüllt und 50 Minuten lang bei 4 bar Druck mit einer Umwälzmenge von 7 m$^3$/h mittels der Pumpe P1 umgewälzt. Der Inhalt des Behälters wurde analysiert und ein Gehalt von 8,7 Gramm Schizophyllan pro Liter ermittelt.

Dann wurde die Umwälzmenge auf 4,1 m$^3$/h eingestellt und ein Transmembrandruck von 1,1 bar angelegt. Die Überströmgeschwindigkeit war 1,7 m/s. Das aus den Filtermodulen austretende Permeat wurde gesammelt und gewogen. 50 Minuten nach dem Beginn der Permeatabnahme wurden 25 kg Fermentationsbrühe in den Behälter B1 zugegeben. 16 Stunden und 20 min nach dem Beginn der Permeatabnahme wurden 40 kg Fermentationsbrühe in den Behälter B1 zugegeben und die Umwälzmenge auf 6,5 m$^3$/h eingestellt. Bis zu diesem Zeitpunkt waren 77 kg Permeat gewonnen worden. Dies entspricht einem mittleren Permeatfluss von 5,6 kg/m2/h. Nach 20 Stunden seit Beginn des Experimentes wurden nochmals 55 kg Fermentationsbrühe in den Behälter B1 zugegeben. Nach 22,5 h nach Beginn des Experimentes hatten sich 109 kg Permeat im Permatbehälter gesammelt. Das Permeat wurde analysiert.

Der MPFR-Wert des Permeats in diesem ersten Filtrationsschritt betrug 1,3. Der Gehalt an Schizophyllan betrug 6,9 Gramm pro Liter (mittlere Rückhaltung bis zu diesem Zeitpunkt 26%) und die Viskosität bei 7/s 1380 mPas.

Nun wurde der Sammelbehälter für das Permeat gewechselt, nochmals 20 kg Fermentationsbrühe in den Behälter B1 zugegeben und die Filtration für weitere 19,5 h betrieben. In dieser Zeit wurde weitere 85 kg Permeat gewonnen. Dies entspricht einem mittleren Permeatfluss von 5,1 kg/h/m2.

Das während des zweiten Filtrationsschrittes gesammelte Permeat wurde analysiert. Der MPFR-Wert betrug 1,2, der Gehalt an Schizophyllan betrug 7,8 Gramm pro Liter (mittlere Rückhaltung über das gesamte Experimente 29%) und die Viskosität bei 7/s 1560 mPas.

Die Ausbeute über beide Filtrationsschritte betrug also 64%. Der Aufkonzentrierungsfaktor war 4,2.

Diskussion

In der nachfolgenden Tabelle 1 sind nochmals die Werte vom Vergleichsbeispiel und vom Beispiel zusammengestellt.

Tabelle 1

| | Vergleichsbeispiel | Beispiel 1 | |
|---|---|---|---|
| | | 1. Stufe | 2. Stufe |
| MPFR-Wert | 2,8 | 1,3 | 1,2 |
| Rückhaltung | 32% | 26% | 29% |
| Aufkonzentrierungsfaktor | 1,07 | 4,2 | |
| Ausbeute | 4 % | 64 % | |

Die Versuche zeigen, dass das erfindungsgemäß filtrierte Produkt wesentlich weniger Bestandteile enthält, welche den 1,2 μm- Filter bei der Bestimmung des MPFR-Wertes verstopfen können. Mit dem erfindungsgemäßen Verfahren kann die Fermentationsbrühe viel stärker aufkonzentriert werden. Die Ausbeute beim erfindungsgemäßen Verfahren ist deutlich höher, und außerdem ist die Rückhaltung beim erfindungsgemäßen Beispiel mit asymmetrischen Filtermembranendeutlich niedriger als bei Vergleich mit symmetrischen Filtermembranen.

Erfindungsgemäßes Beispiel 2

Filtration mit einer asymmetrischen Filtermembran

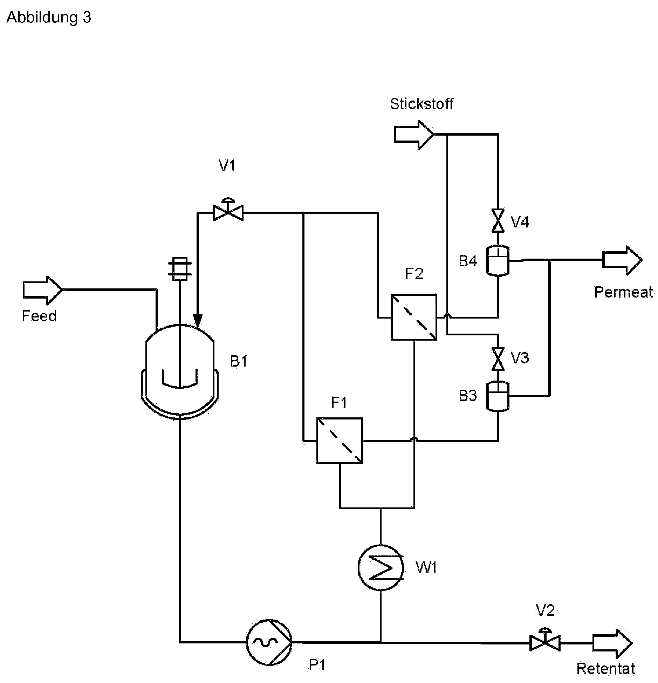

Es wurde wiederum die im Beispiel 1 beschriebene Querstrom-Filtrationsapparatur verwendet. Allerdings war die Apparatur zur Permeatrückspülung mit zwei Kolbensystemen "BACKPULSE DECOLMATEUR BF 100" ausgestattet (siehe Abbildung 3, Positionen B3 und B4). Die Filtermodule F1 und F2 wurden mittels der Kugelhähne V3 und V4 mit Permeat im Abstand von jeweils 900 s mit jeweils 100 ml Permeat zurückgespült, der Druck des Stickstoffes betrug 10 bar.

Über den Doppelmantel des Behälters B1 und den Wärmetauscher W1 wurde der Inhalt der Querstromfiltrationsanlage auf 29°C bis 30°C temperiert.

In den Filtermodulen F1 und F2 wurde eine asymmetrische Rohrmembran aus Aluminiumoxid eingesetzt, und zwar ein 19-Kanal-Element (Modell "MEMBRALOX, Typ EP 1940" der Fa. Pall). Die Porengröße D90 der Membranen

betrug 5,0 μm. Die Porengröße D90 des Trägermaterials betrug 12 μm. Die Länge des Membranrohres war 1020 mm. Das Membranrohr besitzt die Form eines Sechsecks mit abgerundeten Ecken, wobei der Abstand zwischen zwei gegenüber liegenden Ecken 31 mm und der Abstand zwischen zwei gegenüberliegenden Kanten 28 mm beträgt. Die Membranfläche eines Modulelementes betrug 0,24 m². Der Durchmesser eines Kanals war 4 mm.

Für den Versuche wurde ein wie im Vergleichsbeispiel beschrieben hergestellter Fermentationsaustrag mit einem Gehalt von 8,3 Gramm Schizophyllan pro Liter verwendet. Zu Versuchsbeginn wurden 100 kg dieser Fermentationsbrühe (= Feed) in den Behälter B1 eingefüllt, die Umwälzmenge der Pumpe P1 auf 2,8 m³/h eingestellt und ein Transmembrandruck von 0,9 bar angelegt. Die Überströmgeschwindigkeit war 1,6 m/s. Das aus den Filtermodulen austretende Permeat wurde gesammelt und gewogen. 20 Minuten nach dem Beginn der Permeatabnahme wurden 41 kg Fermentationsbrühe in den Behälter B1 zugegeben. 10 Stunden und 35 min nach dem Beginn der Permeatabnahme war der Transmembrandruck auf 1,8 bar angestiegen. Die Permeatabnahme wurde unterbrochen. Bis zu diesem Zeitpunkt waren 100,6 kg Permeat gewonnen worden. Dies entspricht einem mittleren Permeatfluss von 19,8 kg/m2/h. Das Permeat wurde analysiert. Der MPFR-Wert des Permeates in diesem ersten Filtrationsschritt betrug 1,7. Der Gehalt an Schizophyllan betrug 6,3 Gramm pro Liter.

Nun wurde der Permeatsammelbehälter gewechselt, weitere 107 kg Fermentationsbrühe in den Behälter B1 zugegeben und der Transmembrandruck auf 1,2 bar eingestellt. Nach 7 Stunden 55 Minuten seit Beginn dieses zweiten Filtrationsschrittes waren 24,3 kg Permeat gewonnen worden. Dies entspricht einem mittleren Permeatfluss von 6,4 kg/m2/h. Die Analyse des Permeates in diesem ersten Filtrationsschritt ergab einen MPFR-Wert von 1,6 und einen Gehalt an Schizophyllan von 7,4 Gramm pro Liter.

Nun wurde der Sammelbehälter für das Permeat gewechselt und die Filtration für weitere 15 Stunden betrieben. In dieser Zeit wurden weitere 47,2 kg Permeat gewonnen, der Transmembrandruck stieg auf 1,5 bar an. Der mittlere Permeatfluss war 6,6 kg/h/m2. Das während des dritten Filtrationsschrittes gesammelte Permeat wurde analysiert. Der MPFR-Wert betrug 2,2, der Gehalt an Schizophyllan 7,7 Gramm pro Liter.

Die Ausbeute an Glucan über die drei Filtrationsschritte war 57%, der Aufkonzentrierungsfaktor 3,3 und die Rückhaltung 28%.

**Patentansprüche**

1. Verfahren zur Herstellung von wässrigen Lösungen von Glucanen mit einer β-1,3-glykosidisch verknüpften Hauptkette sowie β-1,6-glykosidisch daran gebundenen Seitengruppen umfassend die Fermentation von Pilzstämmen, welche Glucane der genannten Struktur sekretieren, in einem wässrigen Nährmedium und anschließender Abtrennung einer wässrigen Lösung des gebildeten Glucans aus der wässrigen, Glucane und Biomasse enthaltenden Fementationsbrühe durch Querstrom-Mikrofiltration, wobei die Konzentration der Glucane in der zu filtrierenden Fermentationsbrühe mindestens 3 g/l beträgt, **dadurch gekennzeichnet, dass** für die Querstrom-Mikrofiltration asymmetrische Filtermembranen umfassend mindestens eine Schicht aus einem Trägermaterial und mindestens ein Trennschicht eingesetzt werden, wobei die Porengröße der Trennschicht 1 μm bis 10 μm und die Porengröße des Trägermaterials 5 μm bis 100 μm beträgt, mit der Maßgabe, dass die Porengröße des Trägermaterials mindestens 1 μm größer ist als die Porengröße der Tennschicht, und wobei die Strömungsgeschwindigkeit der Querströmung 0,2 m/s bis 20 m/s und der Transmembrandruck 0,1 bis 10 bar beträgt.

2. Verfahren gemäß Anspruch 1, **dadurch gekennzeichnet, dass** die Porengröße des Trägermaterials mindestens 5 μm größer ist als die Porengröße der Trennschicht.

3. Verfahren gemäß Anspruch 1 oder 2, **dadurch gekennzeichnet, dass** man die Fermentation bei einer Temperatur von 15 bis 40°C unter Belüftung und Bewegung vornimmt.

4. Verfahren gemäß Anspruch 1, **dadurch gekennzeichnet, dass** es sich bei den Pilzstämmen um *Schizophyllum communeoder Sclerotium rolfsii* handelt.

5. Verfahren gemäß einem der Ansprüche 1 bis 4, **dadurch gekennzeichnet, dass** keramische asymmetrische Filtermembranen eingesetzt werden.

6. Verfahren gemäß einem der Ansprüche 1 bis 4, **dadurch gekennzeichnet, dass** asymmetrische metallische Filtermembranen eingesetzt werden.

7. Verfahren gemäß einem der Ansprüche 1 bis 6, **dadurch gekennzeichnet, dass** als asymmetrische Filtermembranen Mehrkanalelemente eingesetzt werden.

**8.** Verfahren gemäß einem der Ansprüche 1 bis 7, **dadurch gekennzeichnet, dass** man die asymmetrischen Filtermembranen regelmäßig rückspült.

**9.** Verfahren gemäß einem der Ansprüche 1 bis 8, **dadurch gekennzeichnet, dass** man die Fermentation in einer Anlage umfassend mindestens einen Fermentationsbehälter durchführt, der Anlage über einen Seitenstrom Biomasse und Glucan umfassende Fermentationsbrühe entnimmt, hieraus eine wässrige Lösung von Glucanen mittels Querstrom-Mikrofiltration abtrennt, und wobei man zumindest einen Teil der verbleibenden, Biomasse enthaltenden Fermentationsbrühe wieder in den Fermentationsbehälter zurückführt.

**10.** Verfahren gemäß einem der Ansprüche 1 bis 9, **dadurch gekennzeichnet, dass** die Membranen in regelmäßigen Abständen mit einer Mischung aus Wasserstoff-Peroxid und Lauge gereinigt werden, mit der Maßgabe, dass die Reinigung jeweils erfolgt, sobald eine Permeatmenge von 50 kg pro m$^2$ Membranfläche bis zu 5000 kg pro m$^2$ Membranfläche seit der jeweils vorangehenden Reinigung erreicht worden ist.

**11.** Verfahren gemäß einem der Ansprüche 1 bis 10, **dadurch gekennzeichnet, dass** die Membranen in regelmäßigen Abständen mit einer Mischung aus Hypochlorit und Lauge gereinigt werden, mit der Maßgabe, dass die Reinigung jeweils erfolgt, sobald eine Permeatmenge von 50 kg pro m$^2$ Membranfläche bis zu 5000 kg pro m$^2$ Membranfläche seit der jeweils vorangehenden Reinigung erreicht worden ist.

**Claims**

**1.** A process for the preparation of aqueous solutions of glucans having a β-1,3-glycosidically linked main chain and side groups having a β-1,6-glycosidic bond thereto, comprising the fermentation of fungal strains which secrete glucans of said structure, in an aqueous culture medium, and subsequent separation of an aqueous solution of the resulting glucan from the aqueous fermentation broth comprising glucans and biomass by crossflow microfiltration, the concentration of the glucans in the fermentation broth to be filtered being at least 3 g/l, wherein asymmetrical filter membranes comprising at least one layer of a support material and at least one separating layer are used for the crossflow microfiltration, the pore size of the separating layer being from 1 μm to 10 μm and the pore size of the support material being from 5 μm to 100 μm, with the proviso that the pore size of the support material is at least 1 μm greater than the pore size of the separating layer, and the flow velocity of the crossflow being from 0.2 m/s to 20 m/s and the transmembrane pressure being from 0.1 to 10 bar.

**2.** The process according to claim 1, wherein the pore size of the support material is at least 5 μm greater than the pore size of the separating layer.

**3.** The process according to claim 1 or 2, wherein the fermentation is carried out at a temperature of from 15 to 40°C with aeration and movement.

**4.** The method according to claim 1, wherein the fungal strains are *Schizophyllum commune* or *Sclerotium rolfsii.*

**5.** The process according to any of claims 1 to 4, wherein ceramic asymmetrical filter membranes are used.

**6.** The process according to any of claims 1 to 4, wherein asymmetrical metallic filter membranes are used.

**7.** The process according to any of claims 1 to 6, wherein multichannel elements are used as asymmetrical filter membranes.

**8.** The process according to any of claims 1 to 7, wherein the asymmetrical filter membranes are regularly backwashed.

**9.** The process according to any of claims 1 to 8, wherein the fermentation is carried out in a plant comprising at least one fermentation container, fermentation broth comprising biomass and glucan is removed from the plant via a side stream, an aqueous solution of glucans is separated off therefrom by means of crossflow microfiltration, at least part of the remaining fermentation broth comprising biomass being recycled to the fermentation container.

**10.** The process according to any of claims 1 to 9, wherein the membranes are cleaned at regular intervals with a mixture of hydrogen peroxide and alkali, with the proviso that the cleaning is effected in each case as soon as an amount of from 50 kg of permeate per m$^2$ membrane area to 5000 kg of permeate per m$^2$ membrane area has been reached since the respective preceding cleaning.

11. The process according to any of claims 1 to 10, wherein the membranes are cleaned at regular intervals with a mixture of hypochlorite and alkali, with the proviso that the cleaning is effected in each case as soon as an amount of from 50 kg of permeate per m$^2$ membrane area to 5000 kg of permeate per m$^2$ membrane area has been reached since the respective preceding cleaning.

**Revendications**

1. Procédé de fabrication de solutions aqueuses de glucanes comprenant une chaîne principale reliée β-1,3-glycosidiquement et des groupes latéraux reliés β-1,6-glycosidiquement à celle-ci, comprenant la fermentation de souches de champignons qui sécrètent des glucanes de la structure indiquée, dans un milieu nutritif aqueux, puis la séparation d'une solution aqueuse du glucane formé à partir du bouillon de fermentation aqueux contenant des glucanes et une biomasse par microfiltration à courant transversal, la concentration des glucanes dans le bouillon de fermentation à filtrer étant d'au moins 3 g/l, **caractérisé en ce que** des membranes de filtration asymétriques comprenant au moins une couche en un matériau support et au moins une couche de séparation sont utilisées pour la microfiltration à courant transversal, la taille de pores de la couche de séparation étant de 1 μm à 10 μm et la taille de pores du matériau support étant de 5 μm à 100 μm, à condition que la taille de pores du matériau support soit au moins 1 μm plus grande que la taille de pores de la couche de séparation, et la vitesse d'écoulement de l'écoulement transversal étant de 0,2 m/s à 20 m/s et la pression transmembranaire étant de 0,1 à 10 bar.

2. Procédé selon la revendication 1, **caractérisé en ce que** la taille de pores du matériau support est au moins 5 μm plus grande que la taille de pores de la couche de séparation.

3. Procédé selon la revendication 1 ou 2, **caractérisé en ce que** la fermentation est réalisée à une température de 15 à 40 °C avec aération et agitation.

4. Procédé selon la revendication 1, **caractérisé en ce que** les souches de champignons sont *Schizophyllum commune* ou *Sclerotium rolfsii.*

5. Procédé selon l'une quelconque des revendications 1 à 4, **caractérisé en ce que** des membranes de filtration asymétriques céramiques sont utilisées.

6. Procédé selon l'une quelconque des revendications 1 à 4, **caractérisé en ce que** des membranes de filtration asymétriques métalliques sont utilisées.

7. Procédé selon l'une quelconque des revendications 1 à 6, **caractérisé en ce que** des éléments à plusieurs canaux sont utilisés en tant que membranes de filtration asymétriques.

8. Procédé selon l'une quelconque des revendications 1 à 7, **caractérisé en ce que** les membranes de filtration asymétriques sont régulièrement rincées par inversion de courant.

9. Procédé selon l'une quelconque des revendications 1 à 8, **caractérisé en ce que** la fermentation est réalisée dans une unité comprenant au moins un contenant de fermentation, un bouillon de fermentation comprenant une biomasse et des glucanes étant soutirée de l'unité par un courant latéral, une solution aqueuse de glycanes en étant séparée par microfiltration à courant transversal, et au moins une partie du bouillon de fermentation restant contenant la biomasse étant recyclée dans le contenant de fermentation.

10. Procédé selon l'une quelconque des revendications 1 à 9, **caractérisé en ce que** les membranes sont nettoyées à intervalles réguliers avec un mélange de peroxyde d'hydrogène et de lessive, à condition que le nettoyage ait à chaque fois lieu dès qu'une quantité de perméat de 50 kg par m$^2$ de surface de membrane à 5 000 kg par m$^2$ de surface de membrane a été atteinte depuis le nettoyage précédent.

11. Procédé selon l'une quelconque des revendications 1 à 10, **caractérisé en ce que** les membranes sont nettoyées à intervalles réguliers avec un mélange d'hypochlorite et de lessive, à condition que le nettoyage ait à chaque fois lieu dès qu'une quantité de perméat de 50 kg par m$^2$ de surface de membrane à 5 000 kg par m$^2$ de surface de membrane a été atteinte depuis le nettoyage précédent.

Abbildung 1

F

Permeat

W

Feed

P

Retentat

Abbildung 2

Stickstoff

V1

V4

Feed

B1

F2

Permeat

F1

V3

W1

V2

P1

Retentat

Abbildung 3

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 271907 A2
- EP 504673 A1
- DE 4012238 A1
- WO 03016545 A2

**In der Beschreibung aufgeführte Nicht-Patentliteratur**

- Biosynthese, Produktion und Eigenschaften von extrazellulären Pilz-Glucanen. **UDO RAU.** Habilitationsschrift. Technische Universität Braunschweig, 1997, 70-95
- **UDO RAU.** Biopolymers. Verlag WILEY-VCH, 2002, vol. 6, 63-79
- *GIT Fachzeitung Labor,* vol. 12/92, 1233-1238
- Biosynthese, Produktion und Eigenschaften von extrazellulären Pilz-Glucanen. **UDO RAU.** Habilitationsschrift. Technische Universität Braunschweig, 1997
- **BEISPIEL.** Melin, Rautenbach, Membranverfahren. Springer-Verlag, 2007, 309-366
- **MELIN.** Rautenbach, Membranverfahren. Springer-Verlag, 2007, 51-52
- **UDO RAU.** Biopolymers. Verlag WILEY-VCH, vol. 6, 63-79